# EUROPEAN PATENT SPECIFICATION

(11) **EP 0 457 386 B1**
(45) Date of publication and mention of the grant of the patent: **14.12.1994**
(21) Application number: 91201059.2
(22) Date of filing: 03.05.1991
(51) Int. Cl.: C07C 209/60, B01J 31/28

(54) **Process for the preparation of a secondary amine and catalysts therefor**
Verfahren zur Herstellung von sekundärem Amin und Katalysatoren dafür
Procédé pour la préparation d'amine secondaire et catalyseurs pour ce procédé

(30) Priority: 14.05.1990 GB 9010784
(43) Date of publication of application: 21.11.1991
(73) Proprietor: SHELL INTERNATIONALE RESEARCH MAATSCHAPPIJ B.V., 2596 HR Den Haag (NL)
(72) Inventor: Drent, Eit, NL-1031 CM Amsterdam (NL); Breed, Anthonius Johannes Maria, NL-1031 CM Amsterdam (NL)

(56) References cited:
- EP-A- 0 039 061
- EP-A- 0 145 191
- EP-A- 0 240 193
- FR-A- 2 211 002
- FR-A- 2 476 641

## Description

The invention relates to a process for the preparation of a secondary amine, i.e. an organic compound comprising at least one secondary amine group. Furthermore the invention relates to a catalyst system suitable for use in the preparation of said secondary amine compound.

It is known that the aminomethylation of olefins is catalyzed by various transition metals (J. Org.Chem., 47, 445 (1982)). The overall aminomethylation reaction can be described as follows:

>C=C< + CO + 2 H₂ + HN< --> >CH-C<-CH₂-N< + H₂O ,

or:

>C=C< + 3CO + H₂O + HN< --> >CH-C<-CH₂ -N< + 2CO₂ ,

if water is used as hydrogen source. The precursor amine should have at least one hydrogen atom attached to the amino nitrogen, and can be ammonia, or a primary or secondary amine. Through consecutive aminomethylation reactions primary, secondary or tertiary amines, or their mixtures can be prepared starting from ammonia, whereas the use of primary amine precursors may produce secondary and/or tertiary amines. Generally, the prior art processes lead to the formation of tertiary amines irrespective of the type of starting amine used. Furthermore, the aminomethylation process is rather liable to the occurrence of side reactions and the formation of unwanted side products, such as aldehyde or formamides.

US 4096150 discloses a process for the preparation of tertiary amines, wherein the catalyst system comprised a combination of on the one hand a Group VIII metal, and on the other hand at least one of a broad group of ligands including phosphites, phosphoramides, ethers, amines, heterocyclic bases, sulphides, and mixed donor atom ligands. Among many examples using various transition metals, Examples 9-12 use a Ru based catalyst, which is a ruthenium complex comprising coordinated chloride atoms.

Some prior attempts have been directed at the preparation of secondary amines. US 4317932 discloses a process for the preparation of secondary amines using rhodium based catalysts. However, quite severe reaction conditions were required, and primary and tertiary amine side product were formed to some extent. Moreover, rhodium catalysts being active hydroformylation catalysts, may tend to form aldehyde side product as shown in the comparative example hereinafter.

In Chem. Ind. (Dekker) 22 (Catal. Org. React.) 381-90 (1985), Jachimowicz et al. recommend the use of mixed Ru/Rh catalysts for the aminomethylation of diene polymers. Though apparently satisfactory amination could be achieved, no quantitative data on selectivity are provided. Moreover, the catalyst systems disclosed suffer from the disadvantage of comprising equivalent amounts of oxidation-sensitive phosphines, rendering them less robust and less readily recoverable under conditions of industrial use. In this connection, EP 240193 proposes the preparation of secondary amines in the presence of a catalyst combination of a ruthenium compound, optionally a rhodium compound and an phosphine, wherein the mole ratio of phosphine to ruthenium is greater than 80 to 1. Such a large excess of phosphine will be beneficial in the recovery of spent catalyst by preventing any plating out of rhodium or ruthenium metal. However, the presence of an excess of phosphine appeared to shift the predominant reaction to the formation of imines, so that a second reaction step using conventional hydrogenation catalysts was required to arrive at the desired secondary amines.

Although the versatility of the aminomethylation reaction is well established in the prior art, there remained to exist a need for a specific catalyst system, which selectivity produces secondary amines and is usable under economic conditions.

Surprisingly, it has now been found that secondary amines can be obtained with a high conversion and selectivity using a particular ruthenium catalyst.

Accordingly the invention provides a process for the preparation of an organic compound containing at least one secondary amine group, which process comprises reacting ammonia or a primary amine with carbon monoxide, a hydrogen source and an olefinically unsaturated compound in the presence of a catalyst system comprising a source of cationic ruthenium, an aromatic N-heterocyclic ligand and a source of an anion other than a halide.

It was further found that in the present catalyst system the ligand may be present in excess relative to ruthenium without adverse effect on the selectivity to the desired secondary amines, whereby the recovery of the catalyst is facilitated. This finding is particularly surprising in view of the contrary behaviour of phosphine ligands as shown in the comparative example hereinafter.

According to a preferred embodiment of the invention, the present catalyst system additionally comprises a source of cationic rhodium, in particular for reactions wherein the substrate olefinically unsaturated compound contains a plurality of olefinic bonds.

The primary amine used in the process according to the invention may be aliphatic or aromatic, for example an alkylamine, a cycloalkylamine or an arylamine.

In this specification, unless otherwise stated, an alkyl group is preferably a C₁₋₂₀ alkyl group, more preferably a C₁₋₁₀ alkyl group, for example a methyl, ethyl, propyl group, or any of the isomeric butyl, pentyl or hexyl groups. A cycloalkyl group is preferably a C₃₋₆ cycloalkyl group, for example a cyclopentyl or cyclohexyl group. An aryl group is preferably a C₆₋₁₀ aryl group such as phenyl or naphthyl.

The alkyl, cycloalkyl or aryl group in an alkylamine, a cycloalkylamine or an arylamine may be substituted or unsubstituted.

Examples of suitable substituents include amino, alkylamino, dialkylamino, acylamino (e.g. alkanoylamino), acyl (e.g. alkanoyl), acyloxy (e.g. alkanoyloxy), carboxy, alkoxycarbonyl, hydroxyl, alkoxy, sulphonyl, alkyl, cycloalkyl and aryl. It will be appreciated that an amino substituent may take part in the reaction.

Examples of primary amines are propylamine, n-butylamine, 1,2-diaminoethane, H₂N-CH₂-CH₂CH₂-N(CH₃)₂, ethanolamine, methylamine, ethylamine, tert-butylamines, sec-butylamine, cyclopentylamine, cyclohexylamine, aniline,

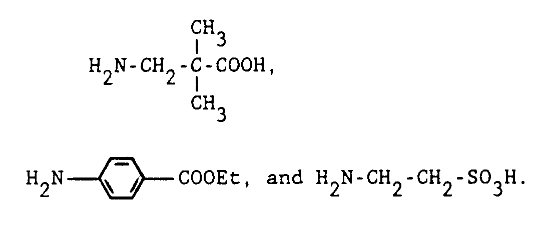

The olefinically unsaturated compound used in the process according to the invention is an olefinic compound containing from 2 to 100 carbon atoms, more preferably from 2 to 30 carbon atoms.

The olefinically unsaturated compound may contain 1, 2 or more olefinic bonds, as for example in an alkene, a cycloalkene, an alkadiene and a cycloalkadiene. An alkene or alkadiene may contain terminal or internal double bonds. Preferably, the olefinic bonds in the olefinically unsaturated compound are not conjugated.

The olefinically unsaturated compound may be substituted or unsubstituted. Examples of suitable substituents include alkyl groups, cycloalkyl groups, aryl groups, dialkylamino groups, acyl groups (e.g. alkanoyl), arylamino groups (e.g. alkanoylamino), aryloxy groups (e.g. alkanoyloxy), alkoxycarbonyl groups, hydroxyl groups, alkoxy groups, acyl groups and acyloxy groups.

Examples of unsubstituted alkenes are ethene, propene, butene, isobutene, the isomeric pentenes or hexenes, 1-octene, diisobutylene, and of substituted alkenes are styrene, oleic acid, and 10-undecenoic acid. Examples of cycloalkenes are cyclohexene and norbornene.

Examples of alkadienes are 1,5-hexadiene, 1,7-octadiene and 1,9-undecadiene. Examples of cycloalkadienes are norbornadiene and dicyclopentadiene.

The process according to the invention is conveniently carried out at a reaction temperature in the range of from 80 to 250 °C, preferably from 100 to 200 °C, more preferably from 110 to 180 °C.

The reaction pressure is conveniently in the range of from 2 to 150 bar, preferably 20 to 100 bar, more preferably 40 to 80 bar. It will be appreciated that when the process is operated batchwise at a constant temperature, the pressure will fall during the course of the reaction.

A source of cationic ruthenium, and, when present, rhodium may be any material comprising ruthenium or rhodium which is capable of yielding cationic ruthenium or rhodium species. Examples of suitable sources include compounds of ruthenium or rhodium such as oxides; salts such as nitrates, sulphates, sulphonates (e.g. trifluoromethanesulphonate or p-toluenesulphonate), phosphates, phosphonates (e.g. benzenephosphonates), carboxylates (e.g. acetates, propionates or butyrates), and beta-carbonyl enolates (e.g. benzoylacetonates or acetylacetonates such as Ru(acac)₃_{,} Rh(acac)(CO)₂); carbonyls (e.g. triruthenium dodecacarbonyl) and hydrides (e.g.H₂Ru₄(CO)₁₃, H₄Ru₄(CO)₁₂, or HRh(CO)(Ph₃P)₃).

The aromatic N-heterocyclic ligand used in the process according to the invention may be monodentate or multidentate containing a plurality of coordinative N-ring atoms. Preferably it is a bidentate ligand containing two coordinative nitrogen atoms. It will be appreciated that the ligand should be inert. The ligand may comprise substituents on the the N-heterocyclic ring or rings, which substituents may be linked together to form condensed ring systems such as in quinoline..

The expression "aromatic N-heterocyclic ligand", as used in the present description, reflects an organic ring compound containing at least one imino N-ring atom and a plurality of double bonds in the ring structure, such that the electron density is at least partly delocalized. The ring may be five, six or seven membered, and may, besides carbon and nitrogen atoms, contain further hetero atoms such as oxygen and sulphur. Two aromatic N-heterocyclic rings may be linked together to form a bidentate ligand such as in 2,2'-bipyridyl or 1,10-phenanthroline.

Examples of aromatic N-heterocyclic ligands are pyridines, e.g. pyridine, 3-methylpyridine, 4-methylpyridine, 2,6-dimethyl-pyridine, 4-ethylpyridine, 2-methoxypyridine, 2-dimethylamino-methylpyridine, 2-pyridinecarboxylic acid; quinolines, e.g. quinoline or 2-methylquinoline; 2,2'-bipyridyls, e.g. 2,2'-bipyridyl, 4,4'-dimethyl-2,2'-bipyridyl, 6,6'-dimethyl, 2,2'-bipyridyl or 4,4'-dicarboxy-2,2'-bipyridyl; 1,10-phenanthrolines, e.g. 1,10-phenanthroline, 4,7-dimethyl-1,10-phenanthroline, 4,7-diphenyl-1,10-phenanthroline disulphonic acid, or 3,4,5,6,7,8-hexamethylphenanthroline. Further suitable ligands include 2-(2-pyridyl)benzimidazole, 3-(2-pyridyl)-5,6-diphenyl-1,2,4-triazine, and the monosodium salt of 3-(2-pyridyl)-5,6-diphenyl-1,2,4-triazine-p,p'-disulphonic acid.

The source of an anion other than a halide may be a salt or an acid. It may also be a metal complex, for example a ruthenium or rhodium complex. The anion is preferably a nitrate, sulphate, sulphonate, phosphate, phosphonate, carboxylate or beta-carbonyl enolate. Preferably the source of anion is a complex or salt of ruthenium or rhodium, or an acid.

The number of moles of aromatic N-heterocyclic ligand per gram atom of ruthenium is preferably in the range of from 0.5 to 1000, more preferably 0.75 to 50, especially 1 to 10.

The number of moles of anion per gram atom of ruthenium is preferably at least 0.5, more preferably in the range of from 1 to 100, especially from 2 to 50.

The number of gram atoms of ruthenium used per mole of olefinically unsaturated compound is not critical. It is preferably in the range of from 10⁻⁶ to 10⁻¹ gram atoms ruthenium per mole of olefinically unsaturated compound.

Where a catalyst system is used which comprises a source of cationic ruthenium and a source of cationic rhodium, the ratio of the number of gram atoms of ruthenium to rhodium is preferably in the range of from 1:100 to 100:1, more preferably 1:10 to 100:1, even more preferably 1:1 to 10:1.

The CO to H₂ ratio is conveniently in the range of from 1:10 to 10:1, preferably 1:5 to 5:1. Most preferably it is 1:2.

The process of the invention may be carried out in the presence or absence of a solvent. Typical examples of solvents are ethers such as diglyme or tetrahydrofuran; alcohols such as hexanol or tetraglycol; esters such as ethyl acetate; ketones such as methyl ethyl ketone; and hydrocarbons such as cyclohexane, toluene and the xylenes.

According to another aspect, the present invention provides a catalyst system which comprises a source of cationic ruthenium, a source of cationic rhodium, an aromatic N-heterocyclic ligand and a source of an anion other than a halide.

The invention will further be illustrated by means of the following not limitative examples. The examples were all carried out in a magnetically stirred 250 ml Hastelloy C autoclave ("Hastelloy" is a trademark). The reaction mixtures obtained were analysed by means of standard gas-liquid chromatography techniques (GLC). The quoted conversions of a reactant represent the proportion (as %) of the converted reactant relative to the amount of reactant charged. The mentioned selectivities represent the proportion (as %) of a selected product relative to the total amount of converted products

### EXAMPLE 1

50 ml diglyme (dimethylether of diethylene glycol), 20 ml 1-octene , 25 ml n-butylamine and as catalyst 0.2 mmol ruthenium acetylacetonate (Ru(acac)₃)/0.3 mmol 2,2'-bipyridyl/2 mmol p-toluenesulphonic acid (pTsa) were introduced into a magnetically stirred 250 ml Hastelloy C autoclave. The autoclave was pressurized to 60 bar with a 1:2 mixture of carbon monoxide and hydrogen, and was then heated to a temperature of 150 °C, which temperature was maintained for 1 hour. The contents of the autoclave were then cooled to the ambient temperature. Product analysis by GLC showed a conversion of 1-octene of 76% with a selectivity of 95% into C₈-CH₂-NH-Bu. Less than 2% conversion of n-butylamine into 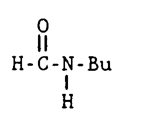
had occurred.

### EXAMPLE 2

50 ml diglyme, 20 ml 1-octene, 25 ml n-butylamine and as catalyst 0.2 mmol Ru(acac)₃/0.6 mmol pyridine/2 mmol pTsa were introduced into a magnetically stirred 250 ml Hastelloy C autoclave. The autoclave was pressurized to 60 bar with a 1:2 mixture of carbon monoxide and hydrogen, and was then heated to a temperature of 150 °C for 1 hour. The contents of the autoclave were then cooled to ambient temperature. Product analysis by GLC showed a conversion of 1-octene of 75% with a selectivity of 95% into C₈-CH₂-NH-Bu. Less than 3% conversion n-butylamine into 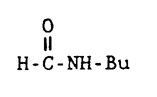
occurred.

### EXAMPLE 3

50 ml diglyme, 20 ml 1-octene, 25 ml n-butylamine and as catalyst 0.2 mmol Ru(acac)₃/0.3 mmol 2,2'-bipyridyl were introduced into a magnetically stirred 250 ml Hastelloy C autoclave. The autoclave was pressurized to 60 bar with a 1:2 mixture of carbon monoxide and hydrogen, and was then heated to a temperature of 150 °C which temperature was maintained for 1.5 hour. The contents of the autoclave were then cooled to ambient temperature. Product analysis by GLC showed a conversion of 1-octene of 70% with a selectivity of 70% into C₈-CH₂-NH-Bu. It appeared that less than 2% of the n-butylamine was converted into the undesired

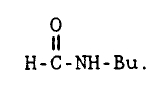

### EXAMPLE 4

50 ml diglyme, 20 ml 1-octene, 25 ml n-butylamine and as catalyst 0.2 mmol Ru(acac)₃/0.3 mmol 2,2'-bipyridyl/2 mmol H₃PO₄ were introduced into a magnetically stirred 250 ml Hastelloy C autoclave. The autoclave was pressurized to 60 bar with a 1:2 mixture of carbon monoxide and hydrogen, and was then heated to a temperature of 130 °C for 5 hours. The contents of the autoclave were then cooled to ambient temperature. Product analysis by GLC showed a conversion of 1-octene of 80% with a selectivity of 85% into C₈-CH₂-NH-Bu. Less than 2% conversion of n-butylamine into 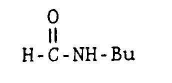
had occurred.

### EXAMPLE 5

50 ml diglyme, 20 ml 1-octene, and as catalyst 0.2 mmol Ru(acac)₃/0.3 mmol 2,2'-bipyridyl/2 mmol pTsa were introduced into a magnetically stirred autoclave. Then ammonia was introduced at a pressure of 4 bar. The autoclave was the pressurized to 60 bar with a 1:2 mixture of carbon monoxide and hydrogen, and was then heated to 150 °C for 5 hours. The contents of the autoclave were then cooled to ambient temperature. Product analysis by GLC showed a conversion of 1-octene of 65% with a selectivity of 60% into (C₈-CH₂)₂-N-H.

### EXAMPLE 6

50 ml diglyme, 20 ml 1-octene, 25 ml n-butylamine and as catalyst 0.2 mmol Ru(acac)₃/0.0025 mmol Rh(acac)(CO)₂/0.3 mmol 2,2'-bipyridyl/2 mmol pTsa were introduced into a magnetically stirred 250 ml Hastelloy C autoclave. The autoclave was pressurized to 60 bar with a 1:2 mixture of carbon monoxide and hydrogen, and was then heated to a temperature of 150 °C for 1.5 hour. The contents of the autoclave were then cooled to ambient temperature. Product analysis by GLC showed a conversion of 1-octene of 95% with a selectivity of 65% into linear C₈-CH₂-NH-Bu. Less than 2% conversion of n-butylamine into 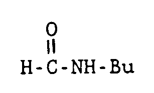
had occurred.

### EXAMPLE 7

50 ml diglyme, 20 ml diisobutylene, 25 ml n-butylamine and as catalyst 0.1 mmol Rh(acac)(CO)₂/0.2 mmol Ru(acac)₃/0.3 mmol 2,2'-bipyridyl/2 mmol pTsa were introduced into a magnetically stirred 250 ml Hastelloy C autoclave. The autoclave was pressurized to 60 bar with a 1:2 mixture of carbon monoxide and hydrogen, and was then heated to a temperature of 140 °C for 5 hours. The contents of the autoclave were then cooled to ambient temperature. Product analysis by GLC showed a conversion of diisobutylene of 60% with a selectivity of 85% into C₈-CH₂-NH-Bu. The conversion into the undesired 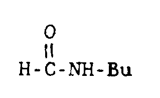
was less than 10%.

### EXAMPLE 8

50 ml diglyme, 20 ml 1-octene, 20 ml H₂N-CH₂-CH₂-CH₂-N(CH₃)₂ and as catalyst the catalyst of Example 5 were introduced into the magnetically stirred 250 ml Hastelloy C autoclave. The autoclave was pressurized to 60 bar with a 1:2 mixture of carbon monoxide and hydrogen, and was then heated to a temperature of 130 °C for 5 hours. The contents of the autoclave were then cooled to ambient temperature. Product analysis by GLC showed a conversion of 1-octene of 91% with a selectivity of 95% into C₈-CH₂-NH-CH₂-CH₂-CH₂-N(CH₃)₂. The linearity of the product was 66%.

### EXAMPLE 9

50 ml hexanol-1 as solvent, 20 ml 1-octene, 20 ml H₂N-CH₂-CH₂-OH and as catalyst the catalyst of Example 5 were introduced into a magnetically stirred 250 ml Hastelloy C autoclave. The autoclave was pressurized to 60 bar with a 1:2 mixture of carbon monoxide and hydrogen, and was then heated to a temperature of 130 °C for 2 hours. The contents of the autoclave were then cooled to ambient temperature. Product analysis by GLC showed a conversion of 1-octene of 85% with a selectivity of 65% into C₈-CH₂-NH-CH₂-CH₂-OH.

### EXAMPLE 10

50 ml diglyme, 10 g oleic acid, 10 ml n-butylamine and as catalyst the same catalyst as in Example 5 were introduced into a magnetically stirred 250 ml Hastelloy C autoclave. The autoclave was pressurized to 60 bar with a 1:2 mixture of carbon monoxide and hydrogen, and was then heated to a temperature of 130 °C, which temperature was maintained for 1 hour. The contents of the autoclave were then cooled to ambient temperature. Product analysis by GLC showed a conversion of oleic acid of 90% with a total selectivity of 90% into N-butylamino-C₁₈-COOH.

### EXAMPLE 11

50 ml diglyme, 10 g 10-undecenoic acid, 20 ml n-butylamine and as catalyst 0.1 mmol Rh(acac)(CO)₂/0.2 mmol Ru(acac)₃/1.2 mmol 2,2'-bipyridyl/2 mmol pTsa were introduced into a magnetically stirred 250 ml Hastelloy C autoclave. The autoclave was pressurized to 60 bar with a 1:2 mixture of carbon monoxide and hydrogen, and was then heated to a temperature of 130 °C, which temperature was maintained for 1 hour. The contents of the autoclave were then cooled to ambient temperature. Product analysis by GLC showed a conversion of 10-undecenoic acid of 80% with a selectivity of 90% into secondary amino acids with a Bu-NH-(CH₂)₁₀-COOH content of 65%.

### EXAMPLE 12

50 ml diglyme, 20 ml 1-octene, 10 g 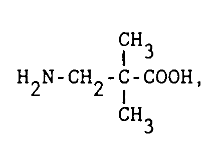
and as catalyst the same catalyst as in Example 10 were introduced into a magnetically stirred 250 ml Hastelloy C autoclave. The autoclave was pressurized to 60 bar with a 1:2 mixture of carbon monoxide and hydrogen, and was then heated to a temperature of 130 °C, which temperature was maintained for 10 hours. The contents of the autoclave were then cooled to ambient temperature. Product analysis by GLC showed a conversion of 1-octene of 50% with a selectivity of 70% into

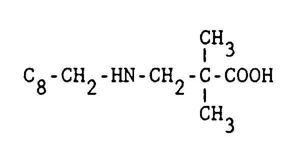

### EXAMPLE 13

50 ml diglyme, 20 ml 1-octene, 10 g 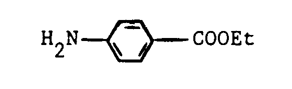
and as catalyst 0.1 mmol Ph(acac)(CO)₂/0.2 mmol Ru(acac)₃/1.2 mmol bipyridyl/2 mmol pTsa were introduced into a magnetically stirred 250 ml Hastelloy C autoclave. The autoclave was pressurized to 60 bar with a 1:2 mixture of carbon monoxide and hydrogen, and was then heated to a temperature of 130 °C, which temperature was maintained for 5 hours. The contents of the autoclave were then cooled to ambient temperature. Product analysis by GLC showed a conversion of 1-octene of 80% with a selectivity of 90% into

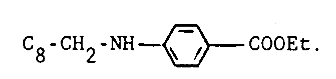

### EXAMPLE 14

50 ml diglyme, 20 g oleic acid, 10 g H₂N-CH₂-CH₂-SO₃H, 145 mmol triethylamine and as catalyst the same catalyst as in Example 12 were introduced into a magnetically stirred 250 ml Hastelloy C autoclave. The autoclave was pressurized to 60 bar with a 1:2 mixture of carbon monoxide and hydrogen, and was then heated to a temperature of 130 °C, which temperature was maintained for 5 hours. The contents of the autoclave were then cooled to ambient temperature. Product analysis by GLC showed a conversion of oleic acid of 100% with a selectivity of 70% into secondary alkylaminosulphonate carboxylic acids and their ammonium salts.

### EXAMPLE 15

50 ml diglyme, 10 ml 1,5-hexadiene, 20 ml n-butylamine and as catalyst 0.2 mmol Ru(acac)₃/0.3 mmol 2,2'-bipyridyl were introduced into a magnetically stirred 250 ml Hastelloy C autoclave. The autoclave was pressurized to 60 bar with a 1:2 mixture of carbon monoxide and hydrogen, and was then heated to a temperature of 150 °C, which temperature was maintained for 5 hours. The contents of the autoclave were then cooled to ambient temperature. Product analysis by GLC showed a conversion of 1,5-hexadiene of 30% with a selectivity of about 10% into N-butylamino disubstituted octanes.

### EXAMPLE 16

50 ml diglyme, 10 ml 1,5-hexadiene, 20 ml n-butylamine and as catalyst 0.1 mmol Rh(acac)(CO)₂/0.2 mmol Ru(acac)₃/0.3 mmol 2,2'-bipyridyl were introduced into a magnetically stirred 250 ml Hastelloy C autoclave. The autoclave was pressurized to 60 bar with a 1:2 mixture of carbon monoxide and hydrogen, and was then heated to a temperature of 130 °C, which temperature was maintained for 5 hours. The contents of the autoclave were then cooled to ambient temperature. Product analysis by GLC showed a conversion of 1,5-hexadiene of 100% with a selectivity of 80% into N-butylamino disubstituted octanes with a content of linear Bu-NH-(CH₂)₈-NH-Bu of 60%.

### EXAMPLE 17

50 ml diglyme, 10 ml 1,7-octadiene, 20 ml n-butylamine and as catalyst the same catalyst as in Example 16 were introduced into a magnetically stirred 250 ml Hastelloy C autoclave. The autoclave was pressurized to 60 bar with a 1:2 mixture of carbon monoxide and hydrogen, and was then heated to a temperature of 130 °C, which temperature was maintained for 2 hours. The contents of the autoclave were then cooled to ambient temperature. Product analysis by GLC showed a conversion of 1,7-octadiene of 100% with a selectivity of 95% into N-butylamino disubstituted decanes with a content of linear Bu-NH-(CH₂)₁₀-NH-Bu of 40%.

### EXAMPLE 18

50 ml diglyme, 7.5 ml 1,5-hexadiene, 20 ml n-butylamine and as catalyst the same catalyst as in Example 16 were introduced into a magnetically stirred 250 ml Hastelloy C autoclave. The autoclave was pressurized to 60 bar with a 1:2 mixture of carbon monoxide and hydrogen, and was then heated to a temperature of 130 °C, which temperature was maintained for 3 hours. The contents of the autoclave were then cooled to ambient temperature. Product analysis by GLC showed a conversion of 1,5-hexadiene of 100% with a selectivity of 65% into N-butylamino disubstituted octanes. The content of the linear Bu-NH-(CH₂)₈-NH-Bu amounted to 60%.

### EXAMPLE 19

50 ml diglyme, 7.5 ml 1,5-hexadiene, 20 ml n-butylamine and as catalyst 0.2 mmol Ru(acac)₃/0.1 mmol Rh(acac)(CO)₂/1.2 mmol 2,2'-bipyridyl were introduced into a magnetically stirred 250 ml Hastelloy C autoclave. The autoclave was pressurized to 60 bar with a 1:2 mixture of carbon monoxide and hydrogen, and was then heated to a temperature of 130 °C, which temperature was maintained for 5 hours. The contents of the autoclave were then cooled to ambient temperature. Product analysis by GLC showed a conversion of 1,5-hexadiene of 100% with a selectivity of 83% into N-butylamino disubstituted octanes. The content of the linear Bu-NH-(CH₂)₈-NH-Bu amounted to 60%.

### EXAMPLE 20

50 ml diglyme, 50 mmol 1,5-hexadiene, 50 mmol H₂N-CH₂-CH₂-NH₂ and as catalyst the same catalyst as in Example 16 were introduced into a magnetically stirred Hastelloy C autoclave. The autoclave was pressurized to 60 bar with a 1:2 mixture of carbon monoxide and hydrogen, and was then heated to a temperature of 130 °C, which temperature was maintained for 5 hours. The contents of the autoclave were then cooled to ambient temperature. Product analysis by GLC showed a conversion of 1,5-hexadiene of 99% into (-NH-CH₂-C₆-CH₂-NH-CH₂CH₂-)ₙ polymer. It appeared that the residual olefin content was 7%.

### Comparative Example A

50 ml diglyme, 20 ml 1-octene, 25 ml n-butylamine and as catalyst 0.2 mmol Ru(acac)₃ were introduced into a magnetically stirred 250 ml Hastelloy C autoclave. The autoclave was pressurized to 60 bar with a 1:2 mixture of carbon monoxide and hydrogen, and was then heated to a temperature of 160 °C, which temperature was maintained for 3 hours. The contents of the autoclave were then cooled to ambient temperature. Product analysis by GLC showed a conversion of 1-octene of as low as 40% with a selectivity of 80% into C₈-CH₂-NH-Bu. It appeared that 50% of the n-butyl amine was converted into the undesired

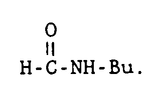

### Comparative Example B

50 ml diglyme, 20 ml 1-octene, 25 ml n-butylamine and as catalyst 0.2 mmol Ru(acac)₃/2 mmol pTsa were introduced into a magnetically stirred 250 ml Hastelloy C autoclave. The autoclave was pressurized to 60 bar with a 1:2 mixture of carbon monoxide and hydrogen, and was then heated to a temperature of 150 °C, which temperature was maintained for 3 hours. The contents of the autoclave were then cooled to ambient temperature. Product analysis by GLC showed a conversion of 1-octene of as low as 30% with a selectivity of 95% into C₈-CH₂-NH-Bu. It appeared that 30% of the n-butyl amine was converted into the undesired 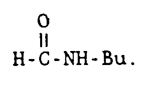
It is seen by comparative examples A and B that in the absence of an aromatic N-heterocyclic ligand, substantial hydroformylation of the amine substrate occurred as an undesired side reaction.

### Comparative Example C

50 ml diglyme, 20 ml 1-octene, 25 ml n-butylamine and as catalyst 0.2 mmol Ru(acac)₃, 0.3 mmol 2,2'-bipyridyl and 2 mmol HCl were introduced into a magnetically stirred 250 ml Hastelloy C autoclave. The autoclave was pressurized to 60 bar with a 1:2 mixture of carbon monoxide and hydrogen, and was then heated to a temperature of 150 °C, which temperature was maintained for 5 hours. The contents of the autoclave were then cooled to ambient temperature. Product analysis by GLC showed a conversion of 1-octene of only about 10%. It appeared that about 30% of the n-butyl amine was converted into the undesired 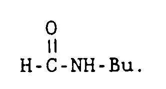
It is seen that high conversion and selectivity are not achieved when the catalyst system comprises halide atoms. By the excess of HCl, the acetylacetonate anions originating from the ruthenium catalyst precursor were converted into nonionic acetylacetone (2,4-pentadione).

### Comparative Example D

50 ml diglyme, 7.5 ml 1,5-hexadiene, 20 ml n-butylamine and as catalyst 0.2 mmol Ru(acac)₃/0.1 mmol Rh(acac)(CO)₂/0.6 mmol triphenyl phosphine were introduced into a magnetically stirred 250 ml Hastelloy C autoclave. The autoclave was pressurized to 60 bar with a 1:2 mixture of carbon monoxide and hydrogen, and was then heated to a temperature of 130 °C, which temperature was maintained for 5 hours. The contents of the autoclave were then cooled to ambient temperature. Product analysis by GLC showed a conversion of 1,5-hexadiene of 100% with a selectivity of 40% to isomeric diamines and a selectivity of 60% to imines and diimines. It is seen that even using small amounts of triphenylphosphine, imines and diimines are formed to a substantial extent, such in conformity with the disclosure of EP 240193.

### Comparative Example E

50 ml diglyme, 20 ml 1-octene, 25 ml n-butylamine and as catalyst 0.1 mmol Rh(acac)(CO)₂ and 0.3 mmol 2,2'-bipyridyl were introduced into a magnetically stirred 250 ml Hastelloy C autoclave. The autoclave was pressurized to 60 bar with a 1:2 mixture of carbon monoxide and hydrogen, and was then heated to a temperature of 165 °C, which temperature was maintained for 5 hours. The contents of the autoclave were then cooled to ambient temperature. Product analysis by GLC showed a conversion of 1-octene of about 100% , but only traces of secondary amine were observed. The main reaction products were imines and aldehydes. It is seen that a rhodium catalyst tends to favour the formation of aldehyde product.

## Claims

1. A process for the preparation of an organic compound having at least one secondary amine group, which process comprises reacting ammonia or a primary amine with carbon monoxide, a hydrogen source and an olefinically unsaturated compound in the presence of a catalyst system comprising a source of cationic ruthenium, an aromatic N-heterocyclic ligand and a source of an anion other than a halide.

2. Process according to claim 1, wherein said primary amine is a C₁₋₂₀ alkyl, C₃₋₆ cycloalkyl or C₆₋₁₀ arylamine.

3. Process according to claim 1 or claim 2, wherein said olefinically unsaturated compound contains from 2 to 30 carbon atoms.

4. Process according to any one of claims 1 to 3, wherein the reaction temperature is in the range of from 100 to 200 °C.

5. Process according to any one of claims 1 to 4, wherein the reaction is carried out at a pressure in the range of from 2 to 100 bar.

6. Process according to any one of claims 1 to 5, wherein the source of cationic ruthenium is an oxide, salt, hydride or carbonyl.

7. Process according to any one of claims 1 to 6, wherein the aromatic N-heterocyclic ligand is an optionally substituted pyridine, quinoline, 2,2'-bipyridyl, or 1,10-phenanthroline.

8. Process according to any one of claims 1 to 6, wherein the aromatic N-heterocyclic ligand is a bidentate ligand containing a plurality of coordinative N-ring atoms.

9. Process according to any one of claims 1 to 8, wherein the anion is a nitrate, sulphate, sulphonate, phosphate, phosphonate, carboxylate or beta-carbonyl enolate.

10. Process according to any one of claims 1 to 9, wherein the number of moles of the aromatic N-heterocyclic ligand per gram atom of ruthenium is in the range of from 1 to 5.

11. Process according to any one of claims 1 to 10, wherein the catalyst system further comprises a source of cationic rhodium.

12. Process according to claim 11, wherein the ratio of the number of gram atoms ruthenium to the number of gram atoms rhodium is in the range of from 1:1 to 10:1.

13. Process according to claim 11 or 12, wherein the olefinically unsaturated compound contains 2 or more olefinic bonds.

14. A catalyst system, which comprises a source of cationic ruthenium, a source of cationic rhodium, an aromatic N-heterocyclic ligand and a source of an anion other than a halide.

15. Catalyst system according to claim 14, wherein the ratio of the number of gram atoms of ruthenium to the number of gram atoms of rhodium is in the range of from 1:10 to 100:1.

16. Catalyst system according to claim 15, wherein said range is from 1:1 to 10:1.

17. Catalyst system according to any one of claims 14 to 16, wherein the source of cationic ruthenium and the source of cationic rhodium is an oxide, salt, hydride or carbonyl.

18. Catalyst system according to any one of claims 14 to 17, wherein the aromatic N-heterocyclic ligand is an optionally substituted pyridine, quinoline, 2,2'-bipyridyl or 1,10-phenanthroline.

19. Catalyst system according to any one of claims 14 to 18, wherein the anion is a nitrate, sulphate, sulphonate, phosphate, phosphonate, carboxylate or beta-carbonyl enolate.

## Patentansprüche

1. Ein Verfahren zur Herstellung einer organischen Verbindung mit mindestens einer sekundären Aminogruppe, welches Verfahren das Umsetzen von Ammoniak oder einem primären Amin mit Kohlenmonoxid, einer Quelle für Wasserstoff und einer olefinisch ungesättigten Verbindung in Anwesenheit eines Katalysatorsystems umfaßt, welches eine Quelle für kationisches Ruthenium, einen aromatischen N-heterocyclischen Liganden und eine Quelle für ein Anion, das kein Halogenid ist, enthält.

2. Verfahren nach Anspruch 1, in dem das primäre Amin ein C₁₋₂₀-Alkylamin, ein C₃₋₆-Cycloalkylamin oder ein C₆₋₁₀-Arylamin ist.

3. Verfahren nach Anspruch 1 oder 2, in dem die olefinisch ungesättigte Verbindung 2 bis 30 Kohlenstoffatome enthält.

4. Verfahren nach irgendeinem der Ansprüche 1 bis 3, in dem die Reaktionstemperatur im Bereich von 100 bis 200°C liegt.

5. Verfahren nach irgendeinem der Ansprüche 1 bis 4, in dem die Reaktion bei einem Druck im Bereich von 2 bis 100 bar durchgeführt wird.

6. Verfahren nach irgendeinem der Ansprüche 1 bis 5, in dem die Quelle für kationisches Ruthenium ein Oxid, ein Salz, ein Hydrid oder ein Carbonyl ist.

7. Verfahren nach irgendeinem der Ansprüche 1 bis 6, in dem der aromatische N-heterocyclische Ligand ein gegebenenfalls substituiertes Pydridin, Chinolin, 2,2'-Bipyridyl oder 9,10-Phenanthrolin ist.

8. Verfahren nach irgendeinem der Ansprüche 1 bis 6, in dem der aromatische N-heterocyclische Ligand ein zweizähniger Ligand ist, der eine Mehrzahl von koordinativ gebundenen N-Ringatomen enthält.

9. Verfahren nach irgendeinem der Ansprüche 1 bis 8, in dem das Anion Nitrat, Sulfat, Sulfonat, Phosphat, Phosphonat, Carboxylat oder beta-Carbonylenolat ist.

10. Verfahren nach irgendeinem der Ansprüche 1 bis 9, in dem die Anzahl Mole an dem aromatischen N-heterocyclischen Ligand je Grammatom Ruthenium im Bereich zwischen 1 bis 5 liegt.

11. Verfahren nach irgendeinem der Ansprüche 1 bis 10, in dem das Katalysatorsystem außerdem eine Quelle für kationisches Rhodium enthält.

12. Verfahren nach Anspruch 11, in dem das Verhältnis der Anzahl an Grammatomen Ruthenium zu der Anzahl an Grammatomen von Rhodium im Bereich von 1:1 bis 10:1 liegt.

13. Verfahren nach einem der Ansprüche 11 oder 12, in dem die olefinisch ungesättigte Verbindung zwei oder mehr olefinische Bindungen enthält.

14. Ein Katalysatorsystem, welches eine Quelle für kationisches Ruthenium, eine Quelle für kationisches Rhodium, einen aromatischen N-heterocyclischen Liganden und eine Quelle für ein Anion enthält, welches kein Halogenid ist.

15. Katalysatorsystem nach Anspruch 14, in dem das Verhältnis der Anzahl von Grammatomen Ruthenium zur Anzahl der Grammatome Rhodium im Bereich von 1:10 bis 100:1 liegt.

16. Katalysatorsystem nach Anspruch 15, in dem besagter Bereich einen Wert von 1:1 bis 10:1 hat.

17. Katalysatorsystem nach irgendeinem der Ansprüche 14 bis 16, in dem die Quelle für kationisches Ruthenium und die Quelle für kationische Rhodium ein Oxid, ein Salz, ein Hydrid oder ein Carbonyl ist.

18. Katalysatorsystem nach irgendeinem der Ansprüche 14 bis 17, in dem der aromatische N-heterocyclische Ligand ein gegebenenfalls substituiertes Pyridin, Chinolin, 2,2-Bipyri-dyl oder ein 1,10-Phenanthrolin ist.

19. Katalysatorsystem nach irgendeinem der Ansprüche 14 bis 18, in dem das Anion ein Nitrat, Sulfat, Sulfonat, Phosphat, Phosphonat, Carboxylat oder beta-Carbonylenolat ist.

## Revendications

1. Un procédé pour la préparation d'un composé organique ayant au moins un groupe amine secondaire, procédé selon lequel on fait réagir de l'ammoniac ou une amine primaire avec du monoxyde de carbone, une source d'hydrogène et un composé à insaturation oléfinique en présence d'un système catalytique renfermant une source de ruthénium cationique, un ligand aromatique N-hétérocyclique et une source d'un anion autre qu'un halogénure.

2. Procédé selon la revendication 1, dans lequel ladite amine primaire est un alkyle en C₁ à C₂₀, un cycloalkyle en C₃ à C₆, ou une arylamine en C₆ à C₁₀.

3. Procédé selon la revendication 1 ou la revendication 2, dans lequel ledit composé à insaturation oléfinique renferme de 2 à 30 atomes de carbone.

4. Procédé selon l'une quelconque des revendications 1 à 3, dans lequel la température de réaction se situe dans la gamme de 100 à 200°C.

5. Procédé selon l'une quelconque des revendications 1 à 4, dans lequel la réaction est mise en oeuvre à une température dans la gamme de 2 à 100 bars.

6. Procédé selon l'une quelconque des revendications 1 à 5, dans lequel la source de ruthénium cationique est un oxyde, un sel, un hydrure ou un carbonyle.

7. Procédé selon l'une quelconque des revendications 1 à 6, dans lequel le ligand aromatique N-hétérocyclique est une pyridine, une quinoléine, un 2,2'-bipyridyle, ou une 1,10-phénanthroline, facultativement substitués.

8. Procédé selon l'une quelconque des revendications 1 à 6, dans lequel le ligand aromatique N-hétérocyclique est un ligand bidenté renfermant une pluralité d'atomes cycliques N, coordinatifs.

9. Procédé selon l'une quelconque des revendications 1 à 8, dans lequel l'anion est un nitrate, un sulfate, un sulfonate, un phosphate, un phosphonate, un carboxylate ou un bêta-carbonyl énolate.

10. Procédé selon l'une quelconque des revendications 1 à 9, dans lequel le nombre de moles du ligand aromatique N-hétérocyclique par atome-gramme de ruthénium se situe dans la gamme de 1 à 5.

11. Procédé selon l'une quelconque des revendications 1 à 10, dans lequel le système catalytique comporte en outre une source de rhodium cationique.

12. Procédé selon la revendication 11, dans lequel le rapport du nombre d'atomes gramme de ruthénium au nombre d'atomes gramme de rhodium se situe dans la gamme de 1:1 à 10:1.

13. Procédé selon la revendication 11 ou la revendication 12, dans lequel le composé à insaturation oléfinique renferme deux ou plus de deux liaisons oléfiniques.

14. Un système catalytique, qui comporte une source de ruthéniun cationique, une source de rhodium cationique, un ligand aromatique N-hétérocyclique et une source d'un anion autre qu'un halogénure.

15. Système catalytique selon la revendication 14, dans lequel le rapport du nombre d'atomes gramme de ruthénium au nombre d'atomes gramme de rhodium se situe dans la gamme de 1:10 à 100:1.

16. Système catalytique selon la revendication 15, dans lequel ladite gamme est de 1:1 à 10:1.

17. Système catalytique selon l'une quelconque des revendications 14 à 16, dans lequel la source de ruthénium cationique et la source de rhodium cationique est un oxyde, un sel, un hydrure ou du carbonyle.

18. Système catalytique selon l'une quelconque des revendications 14 à 17, dans lequel le ligand aromatique N-hétérocyclique est une pyridine, une quinoléine, du 2,2'-bipyridyle ou une 1,10-phénanthroline, éventuellement substitués.

19. Système catalytique selon l'une quelconque des revendications 14 à 18, dans lequel l'anion est un nitrate, un sulfate, un sulfonate, un phosphate, un phosphonate, un carboxylate ou un bêta-carbonylénolate.
